(19) Europäisches Patentamt
European Patent Office
Office européen des brevets

(11) **EP 0 967 475 B1**

(12) **EUROPÄISCHE PATENTSCHRIFT**

(45) Veröffentlichungstag und Bekanntmachung des Hinweises auf die Patenterteilung:
**28.11.2007 Patentblatt 2007/48**

(51) Int Cl.:
*G01N 11/14* (2006.01)    *A61M 1/10* (2006.01)
*F04D 7/04* (2006.01)    *F04D 15/00* (2006.01)

(21) Anmeldenummer: **98810568.0**

(22) Anmeldetag: **22.06.1998**

(54) **Verfahren zur Bestimmung der Viskosität einer Flüssigkeit**

Method for the determination of the viscosity of a liquid

Méthode pour la détermination de la viscosité d' un liquide

(84) Benannte Vertragsstaaten:
**CH DE FR GB LI**

(43) Veröffentlichungstag der Anmeldung:
**29.12.1999 Patentblatt 1999/52**

(73) Patentinhaber: **Levitronix LLC**
**Waltham, MA 02451 (US)**

(72) Erfinder: **Schöb, Reto, Dr.**
**8604 Volketswil (CH)**

(74) Vertreter: **Sulzer Management AG**
**Patentabteilung / 0067**
**Zürcherstrasse 14**
**8401 Winterthur (CH)**

(56) Entgegenhaltungen:
WO-A-97/24596    CH-A- 683 322
DE-A- 19 634 180    US-A- 4 781 525
US-A- 5 725 357

**Beschreibung**

Die Erfindung betrifft ein Verfahren zur Bestimmung der Viskosität einer Flüssigkeit, z.B. von Blut, die von einer Pumpe, z.B. einer Blutpumpe gefördert wird, gemäss dem Oberbegriff des unabhängigen Patentanspruchs.

Pumpen, insbesondere Blutpumpen, die beispielsweise in Herz-Lungen-Maschinen zum Einsatz kommen (als Ersatz für die Pumpfunktion des Herzens), haben einen Einlass und einen Auslass für die zu fördernde Flüssigkeit, hier also das Blut. Weiterhin haben solche Pumpen einen Rotor, der die zu fördernde Flüssigkeit, hier also das Blut, vom Einlass zum Auslass fördert. Speziell bei Blutpumpen, die in Herz-Lungen-Maschinen zum Einsatz kommen, ist eine der Grössen, die ständig überwacht werden müssen, die Viskosität des Bluts. Aber auch bei anderen Pumpen als Blutpumpen kann die Überwachung der Viskosität erforderlich bzw. wünschenswert sein.

In Dokument US 4 781 525 wird ein Verfahren zur Messung des Durchflusses einer Pumpe vorgestellt, in dem auf Grund einer Messung des Drehmomentes T mittels einer empirischen Formel der Form $F = aT + b$ der Durchfluss F bestimmt wird. Zur Kalibrierung der Durchflussmessung wird bei geschlossenem Pumpenausgang ein sogenannter "viscosity calibration factor" bestimmt, der jedoch nicht das für die Viskosität bekannte Verhalten aufweist D.h. die Bestimmung der Viskosität wird umgangen, indem statt dessen ein Korrekturfaktor für eine empirische Durchflussformel bestimmt wird.

In Dokument DE 196 34 180 A1 wird die Bestimmung der Viskosität erwähnt, ohne dass das Vorgehen bei der Bestimmung näher erläutert ist.

Eine Blutpumpe, bei der es möglich ist, die Viskosität des Bluts zu bestimmen, ist beispielsweise aus der US-A-5,725,357 bekannt. Bei der dort beschriebenen Pumpe ist der Rotor in axialer Richtung aktiv magnetisch gelagert. Ein Teil der magnetischen Lagerung wird zwar mit Hilfe von Permanentmagneten gebildet, der andere Teil des magnetischen Lagers wird jedoch mit Hilfe von regelbaren Elektromagneten gebildet, um das aus beiden Teilen gebildete axiale Lager aktiv regeln zu können.

Die Viskositätsbestimmung wird gemäss der DE-A-196 13 388 derart durchgeführt, dass der Regelungsschaltung für das magnetische Lager ein Störsignal mit einer definierten Amplitude und einer definierten Frequenz zugeführt wird, z.B. mit einer Frequenz von 70 Hz. Der Rotor, der sich ohne Störsignal in einer bestimmten Position befindet, wird durch das Störsignal aus dieser Position axial ausgelenkt. Ändert sich nun die Viskosität des Bluts, so ändert sich die axiale Auslenkung des Rotors bei der genannten Störfrequenz von 70 Hz besonders deutlich. Wird dann noch die Abhängigkeit der axialen Auslenkung des Rotors von der jeweiligen Rotordrehzahl bei einer Frequenz des Störsignals von 70 Hz berücksichtigt, so kann über die jeweilige axiale Auslenkung des Rotors die Viskosität des Bluts bestimmt werden.

Diese Blutpumpe bzw. die beschriebene Vorgehensweise ist insofern nachteilig, als eine aktiv regelbare axiale Lagerung des Pumpenrotors vorgesehen sein muss, um das Störsignal über die aktive Lagerung einprägen zu können. Dagegen ist es nicht möglich, die Viskosität auf die oben beschriebene Art und Weise mit Pumpen zu bestimmen, bei denen die axiale Lagerung des Rotors mechanisch ist, weil die mechanische Lagerung des Rotors in axialer Richtung starr ist, sodass eine Auslenkung des Rotors in axialer Richtung nicht stattfinden kann. Auch bei passiven axialen magnetischen Lagerungen, bei denen der Rotor in axialer Richtung durch Reluktanzkräfte gelagert wird, ist eine Bestimmung der Viskosität des Bluts auf diese Weise nicht möglich, weil eine aktive Ansteuerung der Lagerung bei passiven Lagern - die Eigenschaft "passiv" besagt genau dies - eben nicht möglich ist. Somit ist die beschriebene Vorgehensweise nur für Pumpen mit einer aktiven axialen magnetischen Lagerung des Rotors geeignet, für Pumpen mit in axialer Richtung mechanisch gelagerten Rotoren wie auch für Pumpen mit passiven axialen magnetischen Lagerungen ist die beschriebene Vorgehensweise nicht geeignet.

Es ist daher eine Aufgabe der Erfindung, ein Verfahren vorzuschlagen, mit welchem es möglich ist, bei einer Pumpe, z.B. einer Blutpumpe, mit möglichst geringem Aufwand die Viskosität der geförderten Flüssigkeit, z.B. Blut, bestimmen zu können, wobei die Art der Lagerung des Rotors, insbesondere die Art der axialen Lagerung, frei wählbar sein soll. Insbesondere soll das Verfahren natürlich geeignet sein für magnetische Lagerungen des Rotors, die bei Blutpumpen typischerweise zum Einsatz kommen.

Diese Aufgabe wird mit Hilfe eines Verfahrens gelöst, wie es durch die Merkmale des unabhängigen Patentanspruchs charakterisiert ist. Besonders vorteilhafte Varianten des Verfahrens ergeben sich aus den Merkmalen der abhängigen Patentansprüche.

Zur Bestimmung der Viskosität einer Flüssigkeit, z.B. von Blut, die von einer Pumpe, z.B. einer Blutpumpe, gefördert wird, welche Pumpe einen Rotor zur Förderung der Flüssigkeit vom Einlass der Pumpe zum Auslass aufweist, wird ebenfalls der Rotor zur Bestimmung der Viskosität herangezogen. Jedoch erfolgt zur Bestimmung der Viskosität der Flüssigkeit keine aktive Anregung des Rotors zur axialen Auslenkung aus seiner Betriebsposition, sondern der Rotor wird in seiner Betriebsposition belassen und die Viskosität wird aus Messgrössen der Pumpe bzw. des Rotors bestimmt. Somit ist die Art der Lagerung des Rotors, insbesondere die Art der axialen Lagerung des Rotors, frei wählbar. Speziell ist das Verfahren natürlich auch für magnetische Lagerungen des Rotors geeignet, wie sie bei Blutpumpen typischerweise zum Einsatz kommen, insbesondere auch für passive axiale magnetische Lagerungen des Rotors.

Als Messgrössen können das Antriebsmoment bzw. der Antriebsstrom des Rotors und/oder die Rotordrehzahl gemessen werden. Dies sind Grössen, die leicht ausserhalb der Pumpe gemessen werden können. Dies gilt sowohl für das Antriebsmoment des Rotors (oder exakter: des Motors, der den Rotor antreibt), da das Antriebsmoment dem Antriebsstrom näherungsweise direkt proportional ist und für absolute Exaktheit über einen bekannten funktionellen Zusammenhang ermittelt werden kann, als auch für die Drehzahl, die der Spannung am Rotor näherungsweise proportional ist (und für absolute Exaktheit über einen bekannten funktionellen Zusamenhang ermittelt werden kann), sodass für die Praxis die Spannung am Rotor (oder exakter: am Motor, der den Rotor antreibt) als Mass für die Drehzahl gemessen werden kann. Bei drehzahlgeregelten Motoren, das sind Motoren, die auf eine konstante Drehzahl hin geregelt werden, genügt es prinzipiell, nur das Antriebsmoment zu messen (die Drehzahl ist in etwa konstant). Bei momentgeregelten Motoren, das sind Motoren, die auf ein konstantes Antriebsmoment bzw. einen konstanten Antriebsstrom hin geregelt werden, genügt es prinzipiell, nur die Drehzahl zu messen (das Antriebsmoment bzw. der Antriebsstrom ist in etwa konstant). Natürlich ist es immer möglich und natürlich die exakte Vorgehensweise, sowohl die Drehzahl als auch das Antriebsmoment bzw. den Antriebsstrom zu messen. Aus den Werten für Antriebsmoment bzw. Antriebsstrom und/oder Rotordrehzahl wird dann die Viskosität der Flüssigkeit bestimmt.

Dabei können zur Bestimmung der Viskosität mehrere Messungen von Antriebsmoment bzw. Antriebsstrom und/oder Rotordrehzahl durchgeführt werden, sodass mehrere (einzelne) Werte für die Viskosität bestimmt werden. Aus diesen mehreren Werten für die Viskosität wird dann eine mittlere Viskosität bestimmt, welche dann den Wert für die Viskosität darstellt. Dadurch lässt sich die Genauigkeit des Werts für die Viskosität erhöhen.

Weitere Verfahrensvarianten unterscheiden sich dadurch, dass sie entweder bei geschlossenem Aulass der Pumpe durchgeführt werden oder bei geöffnetem Auslass der Pumpe, also quasi "on-line".

Bei einer ersten Reihe von vorteilhaften Verfahrensvarianten wird vor der Bestimmung der Viskosität mit Hilfe der Messgrössen der Pumpenauslass geschlossen. Während einer Herzoperation muss der Auslass operationsbedingt ohnehin mehrmals abgeklemmt werden, sodass die Zeit, in der der Auslass geschlossen ist, für die Bestimmung der Viskosität genutzt werden kann.

Hierbei können die Messungen von Antriebsmoment bzw. Antriebsstrom und/oder Rotordrehzahl durch ein Auslösesignal bewirkt werden, welches nach dem Schliessen des Pumpenauslasses an eine Steuerung weitergeleitet wird, welche dann die Messungen von Antriebsmoment bzw. Antriebsstrom und/oder Rotordrehzahl initiiert.

Ein solches Auslösesignal kann beispielsweise von einer Schliessvorrichtung zum Verschliessen des Pumpenauslasses, z.B. einem Ventil, erzeugt und an die Steuerung geleitet werden, sobald der Pumpenauslass geschlossen ist.

Eine andere Möglichkeit besteht darin, dass das Auslösesignal von einer Durchflussmessvorrichtung, die am Pumpenauslass angeordnet ist, erzeugt und an die Steuerung geleitet wird, sobald kein Durchfluss mehr am Pumpenauslass festgestellt wird. Wenn kein Durchfluss mehr am Pumpenauslass festgestellt wird, bedeutet dies, dass der Pumpenauslass geschlossen ist und die Messungen von Antriebsmoment bzw. Antriebsstrom und/oder Rotordrezahl zur Bestimmung der Viskosität können initiiert werden.

Wieder eine andere Möglichkeit besteht darin, dass das Auslösesignal von einer Druckmessvorrichtung, die am Pumpenauslass angeordnet ist, erzeugt und an die Steuerung geleitet wird, sobald am Pumpenauslass ein Druck festgestellt wird, der bei gegebener Rotordrehzahl einen Schwellenwert überschreitet. Auch das Überschreiten dieses Schwellendrucks bedeutet, dass der Pumpenauslass geschlossen ist und dass die Messungen von Antriebsmoment bzw. Antriebsstrom und/oder Rotordrehzahl zur Bestimmung der Viskosität initiiert werden können.

Weiterhin kann bei einer Pumpe mit Drehzahlregelung ein nach dem Schliessen des Pumpenauslasses auftretender schlagartiger Abfall des Antriebsmoments erkannt und daraufhin das Auslösesignal an die Steuerung geleitet werden. Bei einer Pumpe mit Drehzahlregelung fällt nämlich nach dem Schliessen des Pumpenauslasses das Antriebsmoment bzw. der Antriebsstrom des Rotors schlagartig ab, weil kein Blut mehr gefördert werden kann und das in der Pumpe befindliche Blut sehr schnell die gleiche Geschwindigkeit aufweist wie der Pumpenrotor, sodass zur Aufrechterhaltung dieser Geschwindigkeit nur noch ein geringes Antriebsmoment bzw. ein geringer Antriebsstrom erforderlich ist.

Umgekehrt kann bei einer Pumpe mit Antriebsmomentregelung bzw. Antriebsstromregelung ein nach dem Schliessen des Pumpenauslasses schlagartig auftretender Anstieg der Drehzahl erkannt werden und daraufhin das Auslösesignal an die Steuerung geleitet werden. Bei einer Pumpe mit Antriebsmomentregelung bzw. Antriebsstromregelung steigt nämlich nach dem Schliessen des Pumpenauslasses die Drehzahl sehr schnell an, weil kein Blut mehr gefördert werden kann und das in der Pumpe befindliche Blut sehr schnell die gleiche Drehzahl aufweist wie der Rotor, sodass zur Aufrechterhaltung dieser Geschwindigkeit nur noch ein geringes Antriebsmoment bzw. ein geringer Antriebsstrom erforderlich ist. Gleichwohl steht nach wie vor das gleiche Antriebsmoment bzw. der gleiche Antriebsstrom zur Verfügung wie bei geöffnetem Pumpenauslass, was eine Beschleunigung des Rotors (Drehzahlanstieg) und der Flüssigkeit zur Folge hat.

Bei einer zweiten Reihe von vorteilhaften Verfahrensvarianten werden die Messungen von Antriebsmoment bzw. Antriebsstrom und/oder Rotordrehzahl bei geöffnetem Pumpeneinlass und geöffnetem Pumpenauslass durchgeführt, also quasi "on-line" während des Betriebs. Diese Verfahrensvariante ist zu jeder beliebigen Zeit (z.B. zu jedem beliebigen Zeitpunkt während einer Herzoperation) durchführbar, ausser eben dann, wenn der Pumpenauslass geschlossen ist.

Bei dieser Verfahrensvariante kann die Bestimmung der Viskosität derart erfolgen, dass die Rotordrehzahl um eine Nenndrehzahl herum mit einer Modulationsamplitude und einer Modulationsfrequenz moduliert wird, wobei am Auslass der Pumpe durch die Modulation praktisch keine oder nur eine geringe Änderung des Durchflusses erzeugt wird. Aufgrund der Trägheit der Flüssigkeit erfolgt im wesentlichen keine Durchflussänderung, jedoch wird durch die Modulation eine Schwankung des Antriebsmoments bzw. des Antriebsstroms hervorgerufen. Aus der Modulationsamplitude der Drehzahl und der daraus resultierenden Amplitude der Schwankung des Antriebsmoments bzw. des Antriebsstroms wird dann die Viskosität bestimmt.

Die Bestimmung der Viskosität kann auch derart erfolgen, dass das Antriebsmoment bzw. der Antriebsstrom um ein Nennantriebsmoment bzw. einen Nennantriebstrom herum mit einer Modulationsamplitude und einer Modulationsfrequenz moduliert wird, wobei am Auslass der Pumpe durch die Modulation praktisch keine oder nur eine geringe Änderung des Durchflusses erzeugt wird, jedoch eine Schwankung der Drehzahl hervorgerufen wird.

Auch hier erfolgt also aufgrund der Trägheit der Flüssigkeit im wesentlichen keine Durchflussänderung. Aus der Modulationsamplitude des Antriebsmoments bzw. des Antriebsstroms und der daraus resultierenden Amplitude der Schwankung der Drehzahl wird dann die Viskosität bestimmt.

Da sämtliche vorstehend beschriebenen Verfahrensvarianten speziell auch für Blutpumpen geeignet sind, wird vorteilhafterweise eine Pumpe mit einer magnetischen Lagerung des Rotors verwendet, wie dies bei Blutpumpen (aus mehreren Gründen, z.B. Kontamination) typischerweise der Fall ist.

Nachfolgend wird das erfindungsgemässe Verfahren zur Bestimmung der Viskosität anhand der Zeichnung näher erläutert, in der die beiden vorstehend bereits angesprochenen Ausführungsvarianten - nämlich einerseits bei geschlossenem Ausgang der Pumpe und andererseits bei geöffnetem Ausgang der Pumpe, also quasi "on-line" - dargestellt sind. Es zeigen in schematischer Darstellung:

Fig. 1 eine erste Ausführungsvariante des erfindungsgemässen Verfahrens zur Bestimmung der Viskosität in Form eines Ablaufdiagramms (bei geschlossenem Auslass der Pumpe) und

Fig. 2 eine zweite Ausführungsvariante des erfindungsgemässen Verfahrens zur Bestimmung der Viskosität in Form eines Ablaufdiagramms (bei geöffnetem Auslass der Pumpe, also quasi "on-line").

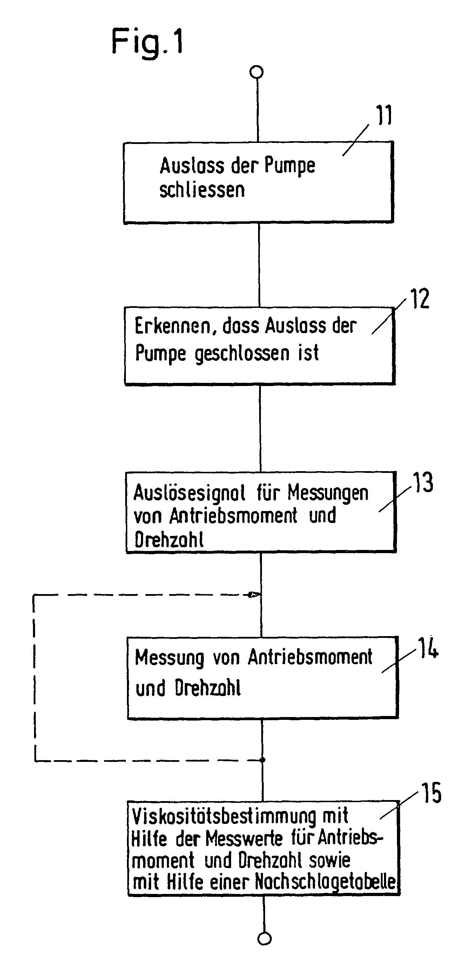

In Fig. 1 ist ein Ablaufdiagramm einer Verfahrensvariante des erfindungsgemässen Verfahrens zur Bestimmung der Viskosität dargestellt.

Nach diesem Ablaufdiagramm können eine erste Reihe von Verfahrensvarianten ablaufen, bei dnen die Bestimmung der Viskosität bei geschlossenem Pumpenauslass erfolgt.

Dazu soll vorausgeschickt werden, dass für das Reibmoment von Flüssigkeiten in Pumpen vereinfacht folgende Beziehung gilt:

$$M_R \sim K_G \cdot \eta \cdot \omega \qquad (I)$$

worin die einzelnen Variablen folgendes bedeuten:

$M_R$    das Reibmoment einer Flüssigkeit,
$K_G$    einen Geometriefaktor,
$\eta$    die dynamische Viskosität der Flüssigkeit,
$\omega$    die Kreisfrequenz des Pumpenrotors.

Bei geschlossenem Pumpenauslass wird der Durchfluss durch die Pumpe null, mit anderen Worten heisst dies, dass die gesamte Antriebsleistung in Flüssigkeitsreibung umgesetzt wird, das Antriebsmoment also dem Reibmoment entspricht:

$$M_A = M_R$$

mit

$M_A$  Antriebsmoment
$M_R$  Reibmoment,

oder als Folge von Beziehung (I) folgt, dass die Viskosität dem Antriebsmoment proportional ist:

$$\eta \ \sim \ M_A$$

In der Praxis sind die Verhältnisse in einer Pumpe etwas komplexer (aufgrund von turbulenter Strömung etc.), dennoch gibt es einen eindeutigen funktionellen Zusammenhang zwischen der Viskosität, der Rotordrehzahl (bzw. der Kreisfrequenz) und dem Antriebsmoment:

$$\eta \ = \ f\,(M_A, \omega) \hspace{3cm} (II)$$

Dieser funktionelle Zusammenhang kann für jede Pumpe messtechnisch erfasst werden und z.B. in Form einer Nachschlagetabelle ("look-up-table") beispielsweise im Speicher eines Mikroprozessors abgelegt werden. Bei dieser Ausführungsvariante des erfindungsgemässen Verfahrens ist eine solche Nachschlagetabelle für die jeweilige Pumpe vor dem Betrieb der Pumpe bereits erfasst und im Speicher des Mikroprozessors abgelegt worden.

Gemäss Fig. 1 läuft dieses Ausführungsbeispiel des Verfahrens zur Bestimmung der Viskosität derart ab, dass in einem ersten Schritt 11 mittels einer Schliessvorrichtung der Auslass der Pumpe geschlossen wird. Während einer Herzoperation muss der Auslass einer solchen Pumpe, die z.B. in einer Herz-Lungen-Maschine eingesetzt wird, operationsbedingt ohnehin mehrmals abgeklemmt werden, sodass die Zeit, in der der Auslass geschlossen ist, für die Bestimmung der Viskosität genutzt werden kann.

Ist der Auslass geschlossen, so kann die Messung von Antriebsmoment bzw. Antriebsstrom und/oder Rotordrehzahl mit Hilfe eines Auslösesignals, welches an eine Steuerung geleitet wird, initiiert werden. Bei einer Pumpe mit Drehzahlregelung (also mit einer Regelung auf eine konstante Drehzahl hin) genügt es prinzipiell, wenn das Antriebsmoment bzw. der Antriebsstrom gemessen wird (die Drehzahl ist ja in etwa konstant), bei einer Pumpe mit Regelung des Antriebsmoments bzw. des Antriebsstroms (also mit einer Regelung auf ein konstantes Antriebsmoment bzw. auf einen konstanten Antriebsstrom hin) genügt es prinzipiell, die Drehzahl zu messen (das Antriebsmoment bzw. der Antriebsstrom ist ja konstant). Natürlich ist es möglich und auch exakt, sowohl das Antriebsmoment bzw. den Antriebsstrom als auch die Drehzahl zu messen. Damit ein Auslösesignal zur Initiierung eines Messvorgangs erzeugt werden kann, muss zunächst in einem Schritt 12 erkannt werden, dass der Auslass der Pumpe geschlossen ist. Ein solches Auslösesignal kann beispielsweise von der Schliessvorrichtung selbst, z.B. einem (elektrisch betätigbaren) Ventil der Herz-Lungen-Maschine, erzeugt und an die Steuerung geleitet werden, sobald der Pumpenauslass geschlossen ist.

Eine andere Möglichkeit besteht darin, eine Durchflussmessvorrichtung am Pumpenauslass vorzusehen, welche das Auslösesignal erzeugt und an die Steuerung leitet, sobald kein Durchfluss mehr am Pumpenauslass festgestellt wird. Dies bedeutet nämlich, dass der Pumpenauslass geschlossen ist.

Wieder eine andere Möglichkeit besteht darin, eine Druckmessvorrichtung am Pumpenauslass vorzusehen, welche das Auslösesignal erzeugt und an die Steuerung leitet, sobald am Pumpenauslass ein Druck festgestellt wird, der bei gegebener Rotordrehzahl einen Schwellenwert überschreitet. Das Überschreiten dieses Schwellendrucks bedeutet, dass der Pumpenauslass geschlossen ist.

Weiterhin kann der geschlossene Auslass bei einer Pumpe mit Drehzahlregelung (also bei einer Pumpe, die auf eine konstante Drehzahl hin geregelt ist) durch einen nach dem Schliessen des Pumpenauslasses auftretenden schlagartigen Abfall des Antriebsmoments erkannt und daraufhin das Auslösesignal an die Steuerung geleitet werden. Bei einer Pumpe mit Drehzahlregelung fällt nämlich nach dem Schliessen des Pumpenauslasses das Antriebsmoment bzw. der Antriebsstrom des Rotors schlagartig ab, weil kein Blut mehr gefördert werden kann und das in der Pumpe befindliche Blut sehr schnell die gleiche Drehgeschwindigkeit aufweist wie der Pumpenrotor, sodass zur Aufrechterhaltung dieser Drehgeschwindigkeit nur noch ein geringes Antriebsmoment bzw. ein geringer Antriebsstrom erforderlich ist.

Umgekehrt kann der geschlossene Auslass bei einer Pumpe mit Antriebsmomentregelung bzw. Antriebsstromregelung (also bei einer Pumpe, die auf ein konstantes Antriebsmoment bzw. auf einen konstanten Antriebsstrom hin geregelt ist) durch einen nach dem Schliessen des Pumpenauslasses schlagartig auftretenden Anstieg der Drehzahl erkannt werden und daraufhin das Auslösesignal an die Steuerung geleitet werden. Bei einer Pumpe mit Antriebsmomentregelung bzw. Antriebsstromregelung steigt nämlich nach dem Schliessen des Pumpenauslasses die Drehzahl sehr schnell an, weil kein Blut mehr gefördert werden kann und das in der Pumpe befindliche Blut sehr schnell die gleiche Drehzahl aufweist wie der Rotor, sodass zur Aufrechterhaltung dieser Geschwindigkeit nur noch ein geringes Antriebsmoment bzw. ein geringer Antriebsstrom erforderlich ist. Gleichwohl steht nach wie vor das gleiche Antriebsmoment bzw. der gleiche Antriebsstrom zur Verfügung wie bei geöffnetem Pumpenauslass, was eine Beschleunigung des Rotors (Drehzahlanstieg) und der Flüssigkeit zur Folge hat.

Nachdem der geschlossene Auslass der Pumpe in Schritt 12 erkannt und das Auslösesignal für die Messungen von Antriebsmoment und Rotordrehzahl in Schritt 13 ausgelöst worden ist, wird von der Steuerung die Messung von Antriebsmoment und/oder Drehzahl initiiert.

Die Messung von Antriebsmoment und/oder Rotordrehzahl erfolgt in einem Schritt 14. Das Antriebsmoment kann dabei derart gemessen werden, dass der Antriebsstrom des Motors, der den Pumpenrotor antreibt, gemessen wird. Der Antriebsstrom ist dem Antriebsmoment näherungsweise direkt proportional (siehe oben), weshalb der Antriebsstrom für die Praxis ein direktes Mass für das Antriebsmoment ist und das Antriebsmoment leicht aus dem gemessenen Wert für den Antriebsstrom berechnet werden kann. Die Rotordrehzahl kann entweder direkt mittels einer an sich bekannten Drehzahlmesseinrichtung gemessen werden, oder es kann die Antriebsspannung des Motors, der den Pumpenrotor antreibt, gemessen werden. Die Antriebsspannung ist der Rotordrehzahl näherungsweise direkt proportional (siehe oben), weshalb die Antriebsspannung ein direktes Mass für die Rotordrehzahl ist und die Rotordrehzahl leicht aus der Antriebsspannung berechnet werden kann. Nach Durchführung der Messungen in Schritt 14 liegen also die Werte für das Antriebsmoment und die Rotordrehzahl vor.

Nun wird in einem Schritt 15 mit Hilfe des Mikroprozessors in der Nachschlagetabelle ("look-up-table") nachgesehen, welche Viskosität aus den gemessenen Werten für Antriebsstrom und Drehzahl resultiert. Sind dabei die in Schritt 14 gemessenen Werte für Antriebsmoment und Rotordrehzahl nicht genau in der Nachschlagetabelle im Speicher des Mikroprozessors vorhanden, so kommen übliche Interpolationsverfahren zum Einsatz.

Es können auch mehrere Messungen von Antriebsmoment bzw. Antriebsstrom und Rotordrehzahl durchgeführt werden (siehe gestrichelte Linie in Fig. 1), z.B. während eines Hochlaufs der Pumpe von null auf die Nenndrehzahl. Entsprechend können auch mehrere Werte für die Viskosität bestimmt werden. Aus diesen mehreren Werten für die Viskosität kann dann durch Mittelungsverfahren eine mittlere Viskosität bestimmt werden, die dann den endgültigen Wert für die Viskosität darstellt, der nach Schritt 15 als ermittelte Viskosität ausgegeben wird. Diese Vorgehensweise erhöht die Genauigkeit der Viskositätsbestimmung.

Besonders geeignet ist diese Art der Viskositätsbestimmung für Pumpen mit einer magnetischen Lagerung des Rotors, weil dann der Einfluss von Lagerreibung, der bei mechanischen Lagern berücksichtigt werden muss, nicht auftritt. Pumpen, insbesondere Blutpumpen, mit einer magnetischen Lagerung des Rotors sind beispielsweise in der WO-A-96/31934 beschrieben.

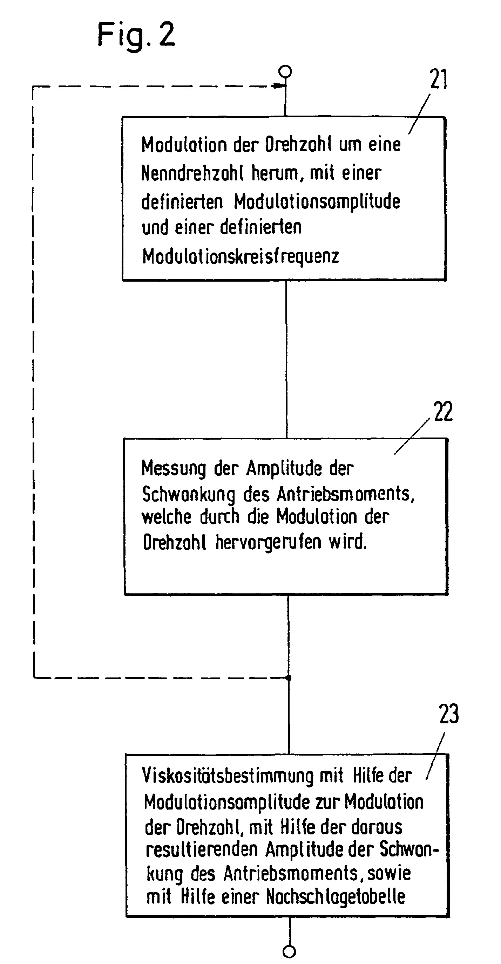

In Fig. 2 ist ein Ablaufdiagramm einer weiteren Ausführungsvariante des erfindungsgemässen Verfahrens zur Bestimmung der Viskosität dargestellt. Diese Ausführungsvariante unterscheidet sich insofern wesentlich von der Ausführungsvariante, die oben anhand von Fig. 1 beschrieben ist, als bei der Ausführungsvariante gemäss Fig. 2 der Auslass der Pumpe nicht geschlossen ist, sondern die Bestimmung der Viskosität bei geöffnetem Pumpenauslass, also quasi "on-line" erfolgt. Nach diesem Ablaufdiagramm können eine zweite Reihe von Verfahrensvarianten ablaufen, bei denen der Pumpenauslass geöffnte ist.

Moduliert man die Nenndrehzahl des Rotors während des Pumpens mit einer genügend hohen Modulations (kreis)frequenz und einer definierten Modulationsamptlitude, so bewirkt die Modulation der Drehzahl aufgrund der Trägheit der Flüssigkeit am Auslass der Pumpe keine Änderung von Druck oder Durchfluss, die Pumpleistung bleibt also praktisch unverändert.

Die Drehzahl lässt sich dabei wie folgt darstellen:

$$n(t) = n_0 + \Delta n \bullet \sin(\omega_m t) \qquad (III)$$

wobei die Variablen folgende Bedeutung haben:

$n(t)$    aktuelle Drehzahl des Rotors
$n_0$    Betriebsnenndrehzahl
$\Delta n$    Modulationsamplitude
$\omega_m$    Modulationskreisfrequenz

Wie bereits erwähnt, wird die Modulationskreisfrequenz so gewählt, dass sich die Modulation nicht in Form von Druck- oder Durchflussschwankungen am Auslass der Pumpe auswirkt. Die durch die Modulation zugeführte Leistung wird vielmehr durch Flüssigkeitsreibung vollständig in Reibungsverluste umgesetzt. Diese Reibungsverluste sind nun aber wieder abhängig von der Viskosität der Flüssigkeit.

Es kann somit ein eindeutiger funktioneller Zusammenhang zwischen der Modulationsamplitude (Drehzahlschwankung), der Amplitude der daraus resultierenden Antriebsmomentschwankung und der Viskosität hergestellt werden. Das Antriebsmoment lässt sich dabei wie folgt darstellen:

$$M_A = M_{A0} + \Delta M_A \bullet \sin(\omega_m t) \qquad (IV)$$

mit

$M_A$     aktuelles Antriebsmoment
$M_{A0}$    mittleres Antriebsmoment
$\Delta M_A$    Amplitude der Antriebsmomentschwankung

Die Amplitude $\Delta M_A$ der Antriebsmomentschwankung hängt nun funktionell mit der Viskosität $\eta$ und der Modulationsamplitude $\Delta n$ der Drehzahl zusammen. Dies lässt sich mathematisch folgendermassen darstellen:

$$\Delta M_A = f(\Delta n, \eta) \qquad (V)$$

bzw.

$$\eta = f(\Delta M_A / \Delta n) \qquad (VI)$$

Die in Gleichung (VI) beschriebene Funktion der Viskosität kann wiederum für jede Pumpe messtechnisch erfasst werden und z.B. in Form einer Nachschlagetabelle ("look-up-table") beispielsweise im Speicher eines Mikroprozessors abgelegt werden. Bei der beschriebenen Ausführungsvariante des erfindungsgemässen Verfahrens ist eine solche Nachschlagetabelle für die jeweilige Pumpe vor dem Betrieb der Pumpe bereits erfasst und im Speicher des Mikroprozessors abgelegt worden.

Gemäss Fig. 2 läuft dieses Ausführungsbeispiel des erfindungsgemässen Verfahrens zur Bestimmung der Viskosität so ab, dass in einem ersten Schritt 21 die Drehzahl des Pumpenrotors um eine Nenndrehzahl $n_0$ herum moduliert wird mit einer definierten Modulationsamplitude $\Delta n$ und einer definierten Modulationskreisfrequenz $\omega_m$.(siehe Gleichung (III)) Die Modulationskreisfrequenz ist dabei so hoch gewählt, dass aufgrund der Trägheit der Flüssigkeit die Drehzahlmodulation am Auslass der Pumpe keine Änderung des Drucks oder des Durchflusses zur Folge hat.

Durch die Modulation der Drehzahl wird eine Schwankung des Antriebsmoments hervorgerufen um ein mittleres Antriebsmoment $M_{A0}$ herum, und zwar mit einer Amplitude $\Delta M_A$ und der gleichen Frequenz $\omega_m$ wie die Modulationskreisfrequenz der Drehzahl (siehe Beziehung (IV)). Diese Amplitude kann in einem Schritt 22 gemessen werden, und zwar als die Amplitude der Schwankung des Antriebsstroms, der dem Antriebsmoment direkt proportional ist, sodass sich aus der Messung der Amplitude der Schwankung des Antriebsstroms die Amplitude der Schwankung des Antriebsmoments leicht berechnen lässt.

Nun wird in einem Schritt 23 mit Hilfe der im Speicher des Mikroprozessors abgelegten Nachschlagetabelle ermittelt, welche Viskosität aus den Werten für die Modulationsamplitude $\Delta n$ der Drehzahl und der Amplitude $\Delta M_A$ der Schwankung des Antriebsmoments resultiert. Sind dabei der in Schritt 22 gemessene Wert für die Amplitude der Schwankung des Antriebsmoments und die Modulationsamplitude für die Drehzahl nicht genau in der Nachschlagetabelle im Speicher des Mikroprozessors vorhanden, so kommen übliche Interpolationsverfahren zum Einsatz.

Bei der beschriebenen Ausführungsvariante können auch mehrere Messungen der Amplitude der Schwankung des Antriebsmoments vorgenommen werden, wobei dann jeweils verschiedene Modulationsamplituden der Drehzahl eingeprägt werden (siehe gestrichelte Linie inFig. 2). Entsprechend können auch mehrere Werte für die Viskosität bestimmt werden. Aus diesen mehreren Werten für die Viskosität kann dann durch Mittelungsverfahren eine mittlere Viskosität bestimmt werden, die dann den endgültigen Wert für die Viskosität darstellt, der nach Schritt 23 als ermittelte Viskosität ausgegeben wird. Diese Vorgehensweise erhöht die Genauigkeit der Viskositätsbestimmung.

Die Funktion in Beziehung (IV) ist - wie man erkennen kann - abhängig von der Modulations(kreis)frequenz $\omega_m$. Für eine Blutpumpe mit einer Nenndrehzahl von ca. 5000 rpm und einem Durchmesser des Rotors von etwa 50 mm kann eine besonders gute Sensitivität ($\Delta M/\Delta\eta$) bei Modulationskreisfrequenzen von etwa 100 rad/s bis etwa 500 rad/s erreicht werden.

Alternativ kann auch das Antriebsmoment moduliert werden und die Amplitude der daraus resultierenden Drehzahlschwankung gemessen werden und diese beiden Werte dann zur Bestimmung der Viskosität herangezogen werden. Die Amplitude der Drehzahlschwankung kann entweder direkt über eine Drehzahlmessung oder über die Spannung am Rotor gemessen werden, die der Drehzahl näherungsweise direkt proportional ist (siehe oben) und daher aus der Spannung am Rotor leicht berechnet werden kann.

Auch diese Art der Viskositätsbestimmung ist speziell für Pumpen mit einer magnetischen Lagerung des Rotors geeignet. Pumpen, insbesondere Blutpumpen, mit einer magnetischen Lagerung des Rotors sind beispielsweise in der WO-A-96/31934 beschrieben.

## Patentansprüche

1. Verfahren zur Bestimmung der Viskosität ($\eta$) einer Flüssigkeit, z.B. von Blut, die von einer Pumpe, z.B. einer Blutpumpe, gefördert wird, welche Pumpe einen Rotor zur Förderung der Flüssigkeit vom Einlass der Pumpe zum Auslass aufweist, wobei der Rotor zur Bestimmung der Viskosität ($\eta$) herangezogen wird, **dadurch gekennzeichnet, dass** zur Bestimmung der Viskosität ($\eta$) der Flüssigkeit keine aktive Anregung des Rotors zur axialen Auslenkung aus seiner Betriebsposition erfolgt, sondern der Rotor in seiner Betriebsposition belassen wird und die Viskosität ($\eta$) aus einem funktionellen, messtechnisch erfassten Zusammenhang zwischen der Viskosität und den Messgrössen der Pumpe bzw. des Rotors bestimmt wird.

2. Verfahren nach Anspruch 1, **dadurch gekennzeichnet, dass** als Messgrössen das Antriebsmoment ($M_A, \Delta M_A$) bzw. der Antriebsstrom des Rotors und/oder die Rotordrehzahl ($n, \Delta n$) gemessen werden, und dass aus den Werten für Antriebsmoment ($M_A, \Delta M_A$) bzw. Antriebsstrom und/oder Rotordrehzahl dann die Viskosität der Flüssigkeit bestimmt wird.

3. Verfahren nach Anspruch 2, **dadurch gekennzeichnet, dass** zur Bestimmung der Viskosität ($\eta$) mehrere Messungen von Antriebsmoment ($M_A, \Delta M_A$) bzw. Antriebsstrom und/oder Rotordrehzahl ($n, \Delta n$) durchgeführt werden, sodass mehrere Werte für die Viskosität bestimmt werden, und dass aus diesen mehreren Werten für die Viskosität eine mittlere Viskosität bestimmt wird, welche dann den Wert für die Viskosität ($\eta$) darstellt.

4. Verfahren nach einem der Ansprüche 1 bis 3, **dadurch gekennzeichnet, dass** vor der Bestimmung der Viskosität ($\eta$) mit Hilfe der Messgrössen der Pumpenauslass geschlossen wird.

5. Verfahren nach Anspruch 4, **dadurch gekennzeichnet, dass** die Messungen von Antriebsmoment ($M_A$) bzw. Antriebsstrom und/oder Rotordrehzahl ($n$) durch ein Auslösesignal bewirkt werden, welches nach dem Schliessen des Pumpenauslasses an eine Steuerung weitergeleitet wird, welche die Messungen von Antriebsmoment ($M_A$) bzw. Antriebsstrom und/oder Rotordrehzahl ($n$) initiiert.

6. Verfahren nach Anspruch 5, **dadurch gekennzeichnet, dass** das Auslösesignal von einer Schliessvorrichtung zum Verschliessen des Pumpenauslasses, z.B. einem Ventil, erzeugt und an die Steuerung geleitet wird, sobald der Pumpenauslass geschlossen ist.

7. Verfahren nach Anspruch 5, **dadurch gekennzeichnet, dass** das Auslösesignal von einer Durchflussmessvorrichtung, die am Pumpenauslass angeordnet ist, erzeugt und an die Steuerung geleitet wird, sobald kein Durchfluss mehr am Pumpenauslass festgestellt wird.

8. Verfahren nach Anspruch 5, **dadurch gekennzeichnet, dass** das Auslösesignal von einer Druckmessvorrichtung, die am Pumpenauslass angeordnet ist, erzeugt und an die Steuerung geleitet wird, sobald am Pumpenauslass ein Druck festgestellt wird, der bei gegebener Rotordrehzahl einen Schwellenwert überschreitet.

9. Verfahren nach Anspruch 5, **dadurch gekennzeichnet, dass** bei einer Pumpe mit Drehzahlregelung ein nach dem Schliessen des Pumpenauslasses auftretender schlagartiger Abfall des Antriebsmoments ($M_A$) bzw. des Antriebsstroms erkannt wird und daraufhin das Auslösesignal an die Steuerung geleitet wird.

**10.** Verfahren nach Anspruch 5, **dadurch gekennzeichnet, dass** bei einer Pumpe mit Antriebsmomentregelung bzw. Antriebsstromregelung ein nach dem Schliessen des Pumpenauslasses auftretender schlagartiger Anstieg der Drehzahl (n) erkannt wird und daraufhin das Auslösesignal an die Steuerung geleitet wird.

**11.** Verfahren nach einem der vorangehenden Ansprüche, wobei der funktionelle, messtechnisch erfasste Zusammenhang zwischen der Viskosität und den Messgrössen der Pumpe bzw. des Rotors in Form einer Nachschlagetabelle ("look-up-table") für die jeweilige Pumpe vor dem Betrieb der Pumpe erfasst und im Speicher eines Mikroprozessors abgelegt wird.

**12.** Verfahren nach einem der Ansprüche 1 bis 3, wobei die Messungen von Antriebsmoment bzw. Antriebsstrom und/ oder Rotordrehzahl bei geöffnetem Pumpeneinlass und geöffnetem Pumpenauslass, also quasi on-line während des Betriebs, durchgeführt werden, und wobei die Rotordrehzahl (n) um eine Nenndrehzahl ($n_0$) herum mit einer Modulationsamplitude ($\Delta n$) und einer Modulationsfrequenz ($\omega_m$) moduliert wird, wodurch eine Schwankung ($\Delta M_A$) des Antriebsmoments bzw. des Antriebsstroms hervorgerufen wird, und dass aus der Modulationsamplitude ($\Delta n$) der Drehzahl und der daraus resultierenden Amplitude ($\Delta M_A$) der Schwankung des Antriebsmoments bzw. des Antriebsstroms die Viskosität ($\eta$) bestimmt wird.

**13.** Verfahren nach einem der Ansprüche 1 bis 3, wobei die Messungen von Antriebsmoment bzw. Antriebsstrom und/ oder Rotordrehzahl bei geöffnetem Pumpeneinlass und geöffnetem Pumpenauslass, also quasi on-line während des Betriebs, durchgeführt werden, und wobei das Antriebsmoment ($M_A$) bzw. der Antriebsstrom um ein Nennantriebsmoment ($M_{A0}$) bzw. einen Nennantriebsstrom herum mit einer Modulationsamplitude ($\Delta M_A$) und einer Modulationsfrequenz ($\omega_m$) moduliert wird, wodurch eine Schwankung ($\Delta n$) der Drehzahl hervorgerufen wird, und dass aus der Modulationsamplitude ($\Delta M_A$) des Antriebsmoments bzw. des Antriebsstroms und der daraus resultierenden Amplitude der Schwankung ($\Delta n$) der Drehzahl die Viskosität ($\eta$) bestimmt wird.

**14.** Verfahren nach einem der vorangehenden Ansprüche, **dadurch gekennzeichnet, dass** eine Pumpe mit einer magnetischen Lagerung des Rotors verwendet wird.

**Claims**

**1.** A method for the determination of the viscosity ($\eta$) of a liquid, e.g. of blood, which is forwarded by a pump, e.g. a blood pump, said pump having a rotor for the forwarding of the liquid from the inlet of the pump to the outlet, with the rotor being used for the determination of the viscosity ($\eta$), **characterised in that** for the determination of the viscosity ($\eta$) of the liquid no active excitation of the rotor for the axial deflection from its operating position takes place, but rather the rotor is left in its operating position and the viscosity ($\eta$) is determined from a functional, technically measured relationship between the viscosity and the measurement parameters of the pump or of the rotor respectively.

**2.** A method in accordance with claim 1 **characterised in that** the drive torque ($M_A$, $\Delta M_A$) or the drive current of the rotor and/or the speed of rotation of the rotor (n, $\Delta n$) are measured as measurement parameters; and **in that** the viscosity of the liquid is then determined from the values for the drive torque ($M_A$, $\Delta M_A$) or the drive current of the rotor and/or for the speed of rotation of the rotor (n, $\Delta n$).

**3.** A method in accordance with claim 2 **characterised in that** for the determination of the viscosity ($\eta$) a plurality of measurements of the drive torque ($M_A$, $\Delta M_A$) or the drive current of the rotor and/or the speed of rotation of the rotor (n, $\Delta n$) are carried out so that a plurality of values for the viscosity are determined; and **in that** from this plurality of values for the viscosity an average viscosity is determined, which then represents the value for the viscosity ($\eta$).

**4.** A method in accordance with one of the claims 1 to 3 **characterised in that** the pump outlet is closed prior to the determination of the viscosity ($\eta$) with the help of the measurement parameters.

**5.** A method in accordance with claim 4 **characterised in that** the measurements of the drive torque ($M_A$) or drive current and/or speed of rotation of the rotor (n) are effected by a trigger signal which is conducted further after the closing of the pump outlet to a control system which initiates the measurements of the drive torque ($M_A$) or drive current and/or the speed of rotation of the rotor (n).

**6.** A method in accordance with claim 5 **characterised in that** the trigger signal is produced by a closure apparatus for the closing of the pump outlet, e.g. by a valve, and is conducted to the control system as soon as the pump outlet is closed.

**7.** A method in accordance with claim 5 **characterised in that** the trigger signal is produced by a through-flow measurement apparatus which is arranged at the pump outlet and is conducted to the control system as soon as no more through-flow is determined at the pump outlet.

**8.** A method in accordance with claim 5 **characterised in that** the trigger signal is produced by a pressure measurement apparatus which is arranged at the pump outlet and is conducted to the control system as soon as a pressure is determined at the pump outlet which exceeds a threshold value at a given speed of rotation of the rotor.

**9.** A method in accordance with claim 5 **characterised in that**, in a pump with regulation of the speed of rotation, an abrupt drop in the drive torque ($M_A$) or of the drive current respectively which arises after the closing of the pump outlet is detected and the trigger signal is thereupon conducted to the control system.

**10.** A method in accordance with claim 5 **characterised in that** in a pump with drive torque regulation or drive current regulation respectively an abrupt rise in the speed of rotation (n) which arises after the closing of the pump outlet is detected and the trigger signal is thereupon conducted to the control system.

**11.** A method in accordance with one of the claims 1 to 3 **characterised in that** the functional, technically measured relationship between the viscosity and the measurement parameters of the pump or of the rotor are determined in the form of a look-up table for the respective pump prior to the operation of the pump and stored in the memory of a microprocessor.

**12.** A method in accordance with one of the claims 1 to 3 wherein the measurements of the drive torque or drive current and/or speed of rotation of the rotor are carried out with the pump inlet open and the pump outlet open, i.e. quasi on-line during operation and wherein the speed of rotation of the rotor (n) is modulated about a nominal speed of rotation ($n_0$) with a modulation amplitude ($\Delta n$) and a modulation frequency ($\omega_m$), through which a fluctuation ($\Delta M_A$) of the drive torque or of the drive current respectively is produced; and in that the viscosity ($\eta$) is determined from the modulation amplitude ($\Delta n$) of the speed of rotation and the amplitude ($\Delta M_A$) of the fluctuation of the drive torque or of the drive current respectively which results therefrom.

**13.** A method in accordance with one of the claims 1 to 3 wherein the measurements of the drive torque or drive current and/or speed of rotation of the rotor are carried out with the pump inlet open and the pump outlet open, i.e. quasi on-line during operation and wherein the drive torque ($M_A$) or the drive current respectively is modulated about a nominal drive torque ($M_{A0}$) or a nominal drive current respectively with a modulation amplitude ($\Delta M_A$) and a modulation frequency ($\omega_m$), through which a fluctuation ($\Delta n$) of the speed of rotation is produced; and in that the viscosity ($\eta$) is determined from the modulation amplitude ($\Delta M_A$) of the drive torque or of the drive current respectively and the amplitude of the fluctuation ($\Delta n$) of the speed of rotation which results therefrom.

**14.** A method in accordance with any one of the preceding claims **characterised in that** a pump with a magnetic journalling of the rotor is used.

**Revendications**

**1.** Procédé pour déterminer la viscosité ($\eta$) d'un liquide, par exemple du sang, qui est transporté d'une pompe, par exemple une pompe à sang, laquelle pompe présente un rotor pour le transport du liquide de l'entrée de la pompe à la sortie, le rotor étant utilisé pour la détermination de la viscosité ($\eta$), **caractérisé en ce que**, pour déterminer la viscosité ($\eta$) du liquide, on n'a pas d'excitation active du rotor pour la déviation axiale à partir de sa position de service, mais le rotor est laissé dans sa position de service et la viscosité ($\eta$) est déterminée à partir d'un rapport fonctionnel, enregistré par les appareils de mesure entre la viscosité et les grandeurs de mesure de la pompe respectivement du rotor.

**2.** Procédé selon la revendication 1, **caractérisé en ce que** le couple d'entraînement ($M_A$, $\Delta M_A$) respectivement le courant d'entraînement du rotor et/ou le régime du rotor (n, $\Delta n$) sont mesurés comme grandeurs de mesure, et **en ce que** la viscosité des liquides est déterminée ensuite à partir des valeurs pour le couple d'entraînement ($M_A$, $\Delta M_A$) respectivement le courant d'entraînement et/ou le régime du rotor (n, $\Delta n$).

**3.** Procédé selon la revendication 2, **caractérisé en ce que**, pour déterminer la viscosité ($\eta$), on effectue plusieurs mesures du couple d'entraînement ($M_A$, $\Delta M_A$) respectivement du courant d'entraînement et/ou du régime du rotor ($n$, $\Delta n$), de sorte que plusieurs valeurs sont déterminées pour la viscosité, et **en ce que**, à partir de ces plusieurs valeurs, on détermine pour la viscosité une viscosité moyenne qui représente alors la valeur pour la viscosité ($\eta$).

**4.** Procédé selon l'une quelconque des revendications 1 à 3, **caractérisé en ce que** la sortie de pompe est fermée avant la détermination de la viscosité ($\eta$) à l'aide des grandeurs de mesure.

**5.** Procédé selon la revendication 4, **caractérisé en ce que** les mesures du couple d'entraînement ($M_A$) respectivement du courant d'entraînement et/ou du régime du rotor ($n$) sont mises en oeuvre par un signal de déclenchement, lequel est transmis après la fermeture de la sortie de pompe à une commande qui amorce les mesures du couple d'entraînement ($M_A$) respectivement du courant d'entraînement et/ou du régime du rotor ($n$).

**6.** Procédé selon la revendication 5, **caractérisé en ce que** le signal de déclenchement est généré par un dispositif de fermeture pour l'obturation de la sortie de pompe, par exemple une vanne, et est dirigé sur la commande dès que la sortie de pompe est fermée.

**7.** Procédé selon la revendication 5, **caractérisé en ce que** le signal de déclenchement est généré par un dispositif de mesure de débit, qui est disposé à la sortie de la pompe, et est dirigé sur la commande dès qu'aucun débit n'est plus constaté sur la sortie de pompe.

**8.** Procédé selon la revendication 5, **caractérisé en ce que** le signal de déclenchement est généré par un dispositif de pression, qui est disposé à la sortie de pompe, et est dirigé sur la commande dès qu'on constate à la sortie de pompe une pression qui dépasse une valeur seuil pour un régime de rotor défini.

**9.** Procédé selon la revendication 5, **caractérisé en ce que**, dans le cas d'une pompe avec réglage du régime, une brusque baisse, apparaissant après la fermeture de la sortie de pompe, du couple d'entraînement ($M_A$) respectivement du courant d'entraînement est détectée et ensuite le signal de déclenchement est dirigé sur la commande .

**10.** Procédé selon la revendication 5, **caractérisé en ce que** dans le cas d'une pompe avec réglage du couple d'entraînement respectivement réglage du courant d'entraînement, une brusque élévation, apparaissant après la fermeture de la sortie de pompe, du (des) régime(s) est détectée et ensuite le signal de déclenchement est dirigé sur la commande .

**11.** Procédé selon l'une quelconque des revendications précédentes, le rapport fonctionnel, détecté par des appareils de mesure, entre la viscosité et les grandeurs de mesure de la pompe respectivement du rotor étant enregistré sous la forme d'un tableau de référence ("look-up-table) pour la pompe concernée avant le fonctionnement de la pompe et est déposé dans la mémoire d'un microprocesseur.

**12.** Procédé selon l'une quelconque des revendications 1 à 3, les mesures du couple d'entraînement respectivement du courant d'entraînement et/ou du régime du rotor étant effectuées avec l'entrée de pompe ouverte et la sortie de pompe ouverte, donc pratiquement en ligne pendant le service, et le (les) régime(s) du rotor étant modulés autour du régime nominal ($n_0$) avec une amplitude de modulation ($\Delta n$) et une fréquence de modulation ($\omega_m$), une variation ($\Delta M_A$) du couple d'entraînement respectivement du courant d'entraînement étant provoquée, et en ce que la viscosité ($\eta$) est déterminée à partir de l'amplitude de modulation ($\Delta n$) du régime et de l'amplitude ($\Delta M_A$) en résultant de la variation du couple d'entraînement respectivement du courant d'entraînement.

**13.** Procédé selon l'une quelconque des revendications 1 à 3, les mesures du couple d'entraînement respectivement du courant d'entraînement et/ou du régime du rotor s'effectuant avec l'entrée de pompe ouverte et la sortie de pompe ouverte, donc pratiquement en ligne pendant le service, et le couple d' entraînement ($M_A$) respectivement le courant d'entraînement étant modulés autour d'un couple d'entraînement ($M_{A0}$) respectivement d'un courant d'entraînement nominal avec une amplitude de modulation ($\Delta M_A$) et une fréquence de modulation ($\omega_m$), de sorte qu'une variation ($\Delta n$) du régime est provoquée, et en ce que la viscosité ($\eta$) est déterminée à partir de l'amplitude de modulation ($\Delta M_A$) du couple d'entraînement ($M_{A0}$) respectivement du courant d'entraînement et de l'amplitude en résultant de la variation ($\Delta n$) du régime.

**14.** Procédé selon l'une quelconque des revendications précédentes, **caractérisé en ce qu'**une pompe est utilisée avec une fixation magnétique du rotor.

EP 0 967 475 B1

# Fig.1

Auslass der Pumpe schliessen — 11

Erkennen, dass Auslass der Pumpe geschlossen ist — 12

Auslösesignal für Messungen von Antriebsmoment und Drehzahl — 13

Messung von Antriebsmoment und Drehzahl — 14

Viskositätsbestimmung mit Hilfe der Messwerte für Antriebsmoment und Drehzahl sowie mit Hilfe einer Nachschlagetabelle — 15

12

# Fig. 2

```
                                              21
  ┌──────────────────────────────┐
  │  Modulation der Drehzahl um eine   │
  │  Nenndrehzahl herum, mit einer     │
  │  definierten Modulationsamplitude  │
  │  und einer definierten             │
  │  Modulationskreisfrequenz          │
  └──────────────────────────────┘

                                              22
  ┌──────────────────────────────┐
  │  Messung der Amplitude der         │
  │  Schwankung des Antriebsmoments,   │
  │  welche durch die Modulation der   │
  │  Drehzahl hervorgerufen wird.      │
  └──────────────────────────────┘

                                              23
  ┌──────────────────────────────┐
  │  Viskositätsbestimmung mit Hilfe der   │
  │  Modulationsamplitude zur Modulation   │
  │  der Drehzahl, mit Hilfe der daraus    │
  │  resultierenden Amplitude der Schwan-  │
  │  kung des Antriebsmoments, sowie       │
  │  mit Hilfe einer Nachschlagetabelle    │
  └──────────────────────────────┘
```

**EP 0 967 475 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4781525 A
- DE 19634180 A1
- US 5725357 A
- DE 19613388 A
- WO 9631934 A